# EUROPÄISCHE PATENTSCHRIFT

(11) **EP 2 226 003 B1**
(45) Veröffentlichungstag und Bekanntmachung des Hinweises auf die Patenterteilung: **06.05.2015**
(21) Anmeldenummer: 09154437.9
(22) Anmeldetag: 05.03.2009
(51) Int. Cl.: G01B 9/02, A61B 5/00, A61B 5/103

(54) **Medizintechnische Bildregistrierung mittels optischer Kohärenztomographie**
Medical image registration by means of optical coherence tomography
Enregistrement d'image médicale à l'aide de la tomographie de cohérence optique

(43) Veröffentlichungstag der Anmeldung: 08.09.2010
(73) Patentinhaber: Brainlab AG, 85622 Feldkirchen (DE)
(72) Erfinder: Urban, Alexander, 85661, Forstinning (DE); Druse, Alexander, 80637, München (DE); Vollmer, Fritz, 80469, München (DE)
(74) Vertreter: Schwabe - Sandmair - Marx

(56) Entgegenhaltungen:
- WO-A-2008/036050
- WO-A-2008/134236
- US-A- 6 006 126
- US-A- 6 167 145

## Beschreibung

Die folgende Erfindung betrifft das technische Gebiet der medizintechnischen Bildregistrierung, bei welcher ein realer Patientenkörperteil positionell einem gespeicherten Bild des Körperteils computerunterstützt zugeordnet wird, um so eine bildgeführte bzw. bildunterstützte Behandlung durchführen zu können.

Es gibt verschiedene Arten, in einem solchen Umfeld einen Patienten zu registrieren, und eine davon umfasst die Verwendung von Markern, die gescannt und während der Behandlung mittels eines medizintechnischen Trackingsystems erfasst werden. Eine solche Registrierungsmethode ist beispielsweise in der DE 196 39 861 A1 beschrieben.

Eine andere Art der Registrierung ist aus der EP 1 142 536 B1 bekannt, und hierbei wird die Oberfläche des Patienten, die mit aufgestrahlten Lichtpunkten erfassbar gemacht wurde, gescannt und einem entsprechenden Oberflächenverlauf in vorab akquirierten Patientenbilddaten zugeordnet.

Aus der WO 2008/134236 A1 ist schließlich noch ein Registrierungssystem bekannt, das mit einem so genannten "Weichgewebe-durchdringenden" Lasersystem arbeitet.

Es ist die Aufgabe der vorliegenden Erfindung, ein Registrierungsverfahren für einen Patienten körperteil zu schaffen, welches eine hochgenaue Registrierung ermöglicht und dabei nicht invasiv bleibt.

Diese Aufgabe wird durch ein medizintechnisches Bildregistrierungsverfahren gemäß dem Anspruch 1 sowie durch eine Pointereinheit eines medizintechnischen Bildregistrierungsgeräts gemäß dem Anspruch 13 gelöst. Die Unteransprüche definieren bevorzugte Ausführungsformen der Erfindung.

Beim erfindungsgemäßen medizintechnischen Bildregistrierungsverfahren wird ein Patientenkörperteil registriert, der eine starre Struktur und eine darüber liegende Weichstruktur aufweist, indem bei der Erfassung der realen Raumposition des Körperteils die Position mindestens eines Referenzpunktes auf der starren Struktur durch die geschlossene Weichstruktur hindurch mittels einer optischen Kohärenztomographie (OCT) ermittelt wird. Mit der optischen Kohärenztomographie ist es möglich, starre Strukturen durch Weichstrukturen hindurch anzumessen, sowie verschiedene Gewebearten und deren Position zu ermitteln. Die vorliegende Erfindung hat den Wert dieser Technologie für die medizintechnische Bildregistrierung erkannt, ebenso wie eine geeignete Umsetzung durch das Anmessen und Lokalisieren von starren Strukturen, die sich unter Weichteilstrukturen befinden.

Die Erfindung könnte in diesem Sinne auch bzw. zusätzlich als Verwendung eines optischen Kohärenztomographen bei der Lokalisierung bzw. Anmessung von Starrstruktur-Referenzpunkten unterhalb einer Weichstruktur angesehen werden.

Der Vorteil des Anmessens bzw. Erfassens von Referenzpunkten auf starren Strukturen liegt darin, dass sie eindeutig zugeordnet werden können und keinen Verschiebungen unterliegen, wie sie z.B. bei aufgeklebten Markern auf Weichgewebe oder bei natürlichen Weichgewebe-Landmarken auftreten können. Auch ist die Genauigkeit des Referenzpunkt-Erfassungsvorganges nicht mehr von der Oberflächenbeschaffenheit des Weichgewebes abhängig, z.B. von dessen Reflektionseigenschaften oder Adsorptionseigenschaften für Licht. Dies alles führt zu einer hochgenauen Registrierung.

Bei einer Ausführungsform der Erfindung wird die reale Raumposition mehrerer Referenzpunkte, einer Wolke von Referenzpunkten, linienförmig aneinander liegender oder beabstandet nebeneinander liegender Referenzpunkte bestimmt, um so ein zuordnungsfähiges Oberflächenabbild zu schaffen.

Die starre Struktur kann eine knöcherne Struktur oder eine Knorpelstruktur sein, während die Weichstruktur eine Hautstruktur sein kann. Diese Beispiele sind aber nicht einschränkend zu verstehen, weil sich eine solche Einschränkung nur durch die Funktionsweise des optischen Kohärenztomographen ergibt. Solange also eine Schicht, die mit dem Kohärenztomographen-Strahl durchdrungen werden kann (hier bezeichnet als Weichgewebe) über eine nicht durchdringungsfähigen, für den Strahl reflektiven Schicht (hier bezeichnet als starre Struktur) liegt, können das vorliegende, erfindungsgemäße Verfahren und die erfindungsgemäße Vorrichtung zum Einsatz kommen. Garantiert wird dabei eine hochgenaue Registrierung, die nicht von der Hautbeschaffenheit oder Lage abhängig ist. Bei der optischen Kohärenztomographie wird monochromatisches Licht verwendet, das eine kurze Kohärenz aufweist und somit die Akquirierung eines detaillierten Tiefenprofils am Patientenkörperteil gestattet. Wenn beispielsweise ein Schädel eines Patienten gescannt wird, um den Scan mit voroperativ akquirierten Daten zu matchen, bringt dies viele Vorteile mit sich, weil die Schädelhautbewegung nicht länger ein Problem ist, ebenso wenig wie das Schwitzen oder die Farbe der Haut. Der knöcherne Schädel entspricht immer den mit einem Bilderzeugungsverfahren (CT, MR, etc.) erfassten präoperativen Daten. Ein weiterer Vorteil liegt darin, dass das Licht, welches verwendet wird, um die Referenzpunkterfassung zu bewirken (OCT-Scan) sehr nahe am infraroten Bereich liegt, so dass für die Sicherheit des Patienten gesorgt ist.

Grundsätzlich können das Verfahren sowie die Pointereinheit gemäß der vorliegenden Erfindung auch verwendet werden, um interoperativ Bilddaten zu erhalten oder zu aktualisieren.

Auf Einzelheiten zur optischen Kohärenztomographie und ihrer erfindungsgemäßen Verwendung wird später bei der Erläuterung des Ausführungsbeispiels noch detaillierter eingegangen, wobei die beschriebenen Eigenschaften natürlich einzeln sowie in jedweder sinnvollen Kombination beim erfindungsgemäßen Verfahren bzw. bei der erfindungsgemäßen Vorrichtung umgesetzt werden können.

Gemäß einer Ausführungsform des Verfahrens der vorliegenden Erfindung wird die Raumpositionserfassung mit Hilfe eines medizintechnischen, optischen Trackingsystems erfolgen und die Registrierung insbesondere durch ein medizintechnisches Navigationssystem durchgeführt werden, dem das Trackingsystem zugeordnet ist. Der OCT-Strahl, d.h. der Scan- oder Abtaststrahl des optischen Kohärenztomographen kann durch den Tomographen selbst oder seine Abstrahleinheit auf das Patientenkörperteil aufgestrahlt werden.

Es ist möglich, die Raumlage des Referenzpunktes auf der starren Struktur zu ermitteln, und zwar durch:
a) die räumliche Erfassung des ihm zugehörigen OCT-Strahl-Reflexpunktes außen auf der Weichstruktur mittels des Trackingsystems, und
b) die Bestimmung der Tiefenlage des Referenzpunktes unter dem Reflexpunkt mittels der optischen Kohärenztomographie.

Zusätzlich oder alternativ kann die genannte Raumlage ermittelt werden durch:
a) die Erfassung der Raumposition des optischen Kohärenztomographen (10) oder seiner Abstrahleinheit, insbesondere durch eine daran angeordnete Referenzeinrichtung bzw. -anordnung (13), mittels des Trackingsystems, und
b) die Bestimmung der Raumlage des Referenzpunktes bezüglich der Raumposition des optischen Kohärenztomographen (10) oder seiner Abstrahleinheit mittels der optischen Kohärenztomographie.

Der optische Kohärenztomograph kann also, muss aber nicht selbst im Trackingsystem verfolgt werden. Er kann bei der Positionsermittlung für den Referenzpunkt mit der Weichstruktur in Kontakt sein oder kontaktlos über der Weichstruktur angeordnet werden. Es gibt auch unterschiedliche, im Rahmen der Erfindung mögliche Registrierung-Zuordnungsarten, wobei bei einer davon eine Zuordnung diskreter Bildwerte erfolgt, insbesondere erfasste Strukturkanten aufeinander registriert werden. Zusätzlich oder alternativ ist es möglich, dass bei der Registrierung der räumlich erfassten Referenzpunkte mit dem gespeicherten Bild eine Grauwert-Zuordnung der Bildwerte erfolgt, die zwar rechnerisch aufwendiger sein dürfte, jedoch eine noch höhere Registrierungsgenauigkeit ermöglicht.

Die erfindungsgemäße Pointereinheit eines medizintechnischen Bildregistrierungsgerätes identifiziert oder definiert einen Referenzpunkt für die Zuordnung bzw. zeigt diesen Referenzpunkt an, und sie umfasst einen optischen Kohärenztomographen (OCT) bzw. seine Abstrahleinheit. Sie kann auch aus dem optischen Kohärenztomographen oder seiner Abstrahleinheit aufgebaut sein, also im Wesentlichen daraus bestehen bzw. ein integriertes Bauteil bilden.

Wie oben schon bei der Erörterung des erfindungsgemäßen Verfahrens angedeutet, kann die Pointereinheit mit einer Referenzeinrichtung bzw. -anordnung versehen sein, die von einem medizintechnischen Trackingsystem identifiziert und/oder positionell erfasst werden kann. Wenn ein geeignetes Referenzpunkt-Erfassungverfahren gewählt wird, ist dies aber nicht unbedingt notwendig.

Es ist möglich, die Pointereinheit mit einer Datenübertragungsvorrichtung auszustatten, um ermittelte Referenzpunkt-Positionsdaten an eine Auswerteeinheit, insbesondere ein zugeordnetes medizintechnisches Navigationssystem zu übermitteln. Die Datenübertragungsvorrichtung kann kabelgebunden oder kabellos (Funk, Bluetooth, etc.) sein.

Bei einer weiteren bevorzugten Ausführungsform weist die Abstrahleinheit des optischen Kohärenztomographen einen Aufsatz auf, insbesondere eine verlängerte Optik speziell eine Lichtleiteroptik, wobei die Lichtleiteroptik im Besonderen ein starrer, länglicher Verlängerungsaufsatz sein kann. Mit einem solchen Aufsatz lassen sich auch Bereiche erreichen und registrieren, die sich an schwer von außen in direkter Sichtlinie zugänglichen Stellen befinden, beispielsweise in der Mundhöhle oder der Nasenhöhle.

Die Erfindung wird im Weiteren anhand von Ausführungsbeispielen näher erläutert. Sie kann alle hierin beschriebenen Merkmale einzeln, sowie in jedweder sinnvollen Kombination umfassen. In den beiliegenden Zeichnungen zeigen:
- Figur 1: eine schematische Darstellung eines OCT-Scans an einem Patientenschädel;
- Figur 2: eine Vergrößerung und schematische Darstellung der OCT-Strahlreflektion; und
- Figuren 3 bis 5: schematische Darstellungen unterschiedlicher Aufbauvarianten für optische Kohärenztomographen.

Zunächst wird hier auf grundsätzliche Eigenschaften der optischen Kohärenztomographie eingegangen, wie sie im Rahmen der vorliegenden Erfindung, d.h. beim erfindungsgemäßen Verfahren, bei der erfindungsgemäßen Pointereinheit oder allgemein bei der Verwendung eines OCT-Gerätes in der Bildregistrierung, umgesetzt werden können. Die im Folgenden beschriebenen Merkmale müssen nicht immer gemeinsam verwirklicht werden, sondern können je nach Ausgestaltung auch alternativ zum Einsatz kommen.

Die erfindungsgemäß einsetzbare optische Kohärenztomographie ist ein zerstörungsfreies, bildgebendes Verfahren, welches die innere Struktur von Materialien darstellen kann. Mittels beispielsweise Infrarotlicht kann kontaktfrei und daher zerstörungsfrei ein Querschnitt einer Probe angefertigt werden, also beispielsweise eines Patientenkörperteils. Die Tiefenauflösung kann bei 20 µm bis 1 µm liegen und das physikalische Prinzip liegt in der interferometrischen Überlagerung von Infrarotlichtwellen, die in unterschiedlicher Probentiefe gestreut werden, mit einer Referenzwelle. Die rückgestrahlte Intensität enthält die Tiefeninformation der Probe und lässt sich nach Anwendung mathematischer Algorithmen als Querschnittsbild darstellen. Mit anderen Worten wird bei der optischen Kohärenztomographie eine Topographie von Oberflächen und Strukturen in streuenden Medien sichtbar gemacht, und zwar mittels eines optischen Interferometers, das die Streustärke und die Tiefenlage der Strukturen mit hoher Auflösung bestimmt.

Die Darstellung von Gewebestrukturen kann z.B. bis zu einer Tiefe von 3 - 8 mm in Form von zweidimensionalen Schnittbildern hindurch bis zur Oberfläche folgen. Das verwendete monochromatische Licht hat eine sehr kurze Kohärenzlänge, und wenn eine Probe mit diesem Licht bestrahlt wird, kommt es zu Reflektionen an der Oberfläche und Streuprozessen in der Probe, die einen Teil des Lichtes zurücksenden. Dieses Probenlicht wird - wie schon erwähnt - mit dem Licht einer Referenzebene überlagert, wobei als Lichtquelle eine SLD mit kurzer Kohärenzlänge eingesetzt wird. Die optische Kohärenztomographie zählt damit zur Kurzkohärenz-Interferometrie.

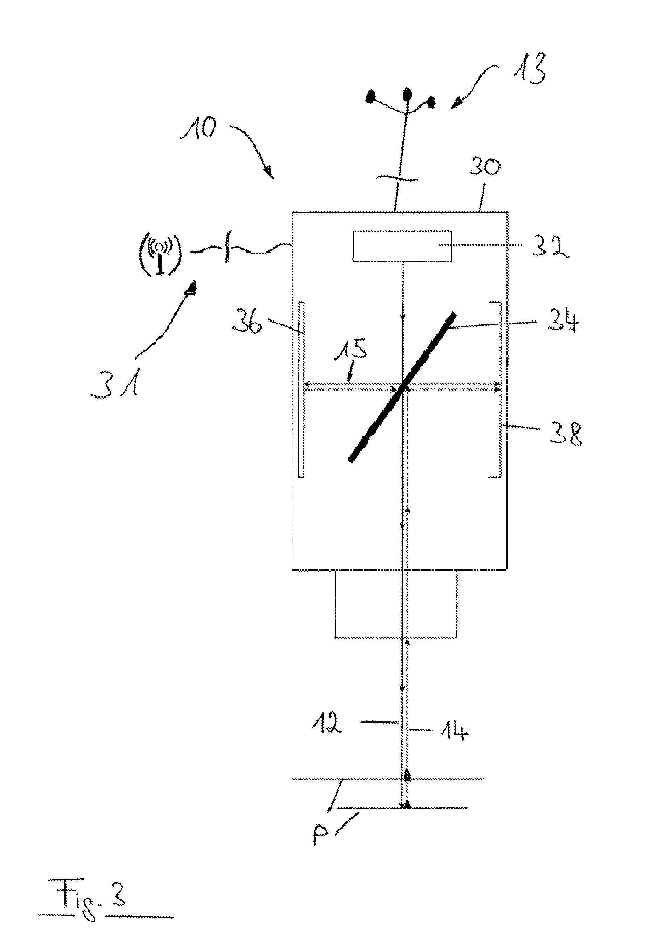

Zur Erläuterung grundsätzlicher Ausgestaltungen optischer Kohärenztomographen wird im Weiteren zunächst auf die Figuren 3 bis 5 Bezug genommen. Der optische Kohärenztomograph ist in diesen drei Figuren insgesamt jeweils mit dem Bezugszeichen 10 gekennzeichnet, und in der Figur 3, die einen grundsätzlichen Aufbau zeigt, sind noch Ausstattungsmerkmale vorhanden, die den Tomographen 10 in ein medizintechnisches Tracking- und Navigationssystem integrieren. Hierzu gehören beispielsweise eine Referenzanordnung 13, die, wie oben schon angedeutet, vorhanden sein kann aber nicht unbedingt notwendig ist (je nach Referenzpunkt-Bestimmungsmethode). Aus diesem Grund ist in der Darstellung die Verbindungslinie durch eine Wellenlinie unterbrochen worden. Ein weiteres solches Merkmal betrifft die Datenanbindung 31, die kabelgebunden oder auch, wie schematisch durch das Funk-Symbol angezeigt, kabellos sein kann. Weil auch hier beide Möglichkeiten bestehen, ist die dargestellte Datenleitung durch eine Wellenlinie unterbrochen.

Bei dem dargestellten, einfachen schematischen Aufbau des Kohärenztomographen 10 der Figur 3 ist eine SLD-Lichterzeugungseinheit vorhanden, d.h. eine Superlumineszenzdiode, die beispielsweise Licht mit einer Wellenlänge von λ= 1280 nm und einer Kohärenzlänge von etwa 20 µm erzeugen kann. Die SLD 32 strahlt ihr Licht durch einen Strahlteiler (z.B. einen halbdurchlässigen Spiegel), und ein Teil, der OCT-Strahl 12, gelangt auf die Probe P und wird aus verschiedenen Ebenen zurückgestrahlt (Strahl 14). Der Strahl 14 trifft dann wieder auf den Strahlteiler 34 und wird von diesem auf den Detektor 38 gelenkt. Der Referenzstrahl 15 geht vom Strahlteiler aus direkt auf einen Spiegel 36 und durch den Strahlteiler 34 hindurch auch zum Detektor 38. Das rückgestrahlte Licht des OCT-Strahls 12, 14 wird also mit dem Licht des Referenzstrahls 15 überlagert. Ein Interferenzsignal entsteht dann, wenn sich die Wellenzüge des rückgestrahlten Lichts mit denen des Referenzstrahls innerhalb der Kohärenzlänge überlagern. Mehrfach gestreutes Licht hat durch die größere Wegdifferenz keine definierte Phasenbeziehung zum Licht des Referenzstrahls und trägt deshalb nicht zum Interferenzsignal bei. Dadurch wird nur einfach gestreutes Licht aus einer definierten Gewebetiefe verarbeitet. Je kürzer die Kohärenzlänge, desto besser ist das Auflösungsvermögen des Verfahrens. Die Darstellung eines Tiefenprofils wird möglich, weil alle Lichtwellen eliminiert werden, die das einfach gestreute Licht verdecken. Das Interferenzsignal ist ein Maß für die Stärke der Streuung in der entsprechenden Gewebetiefe. Die Einstrahltiefe wird durch die Streueigenschaften des Gewebes begrenzt.

Durch die kontinuierliche Variation der Referenzstrahllänge entsteht somit ein Tiefenprofil der Rückstreuintensität an einer Stelle des Gewebes. Durch die laterale Ablenkung des Strahls über die Gewebeoberfläche entstehen zweidimensionale Schnittbilder.

Die am Detektor empfangenen Daten können dann zur Auswertung über die Datenleitung 31 versendet werden oder durch eine eingebaute Auswerteeinheit ausgewertet und als fertige Tiefen- bzw. Schnittbilddaten weitergegeben werden.

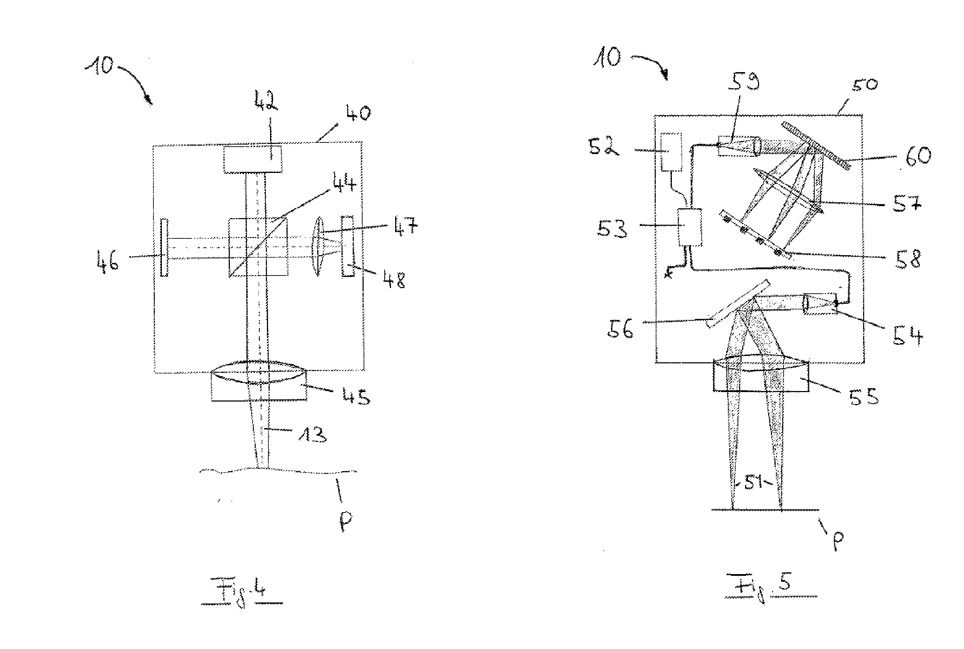

Der Kohärenztomograph 10 der Figur 4 unterscheidet sich in seinem Aufbau dadurch, dass die SLD 42 im Gehäuse 40 einen breiten Strahl 13 durch den Strahlteiler 44 aussendet, der durch eine Linse 45 gebündelt wird, bevor er auf die Probe auftritt. Der durch Spiegel 46 reflektierte Referenzstrahl und der zurückgeworfene OCT-Strahl 13 werden ebenfalls durch eine Linse 47 gebündelt, bevor sie auf den Detektor 48 auftreffen. Durch eine Bewegung des Spiegel 46 in der Horizontalen von links nach rechts kann die Eindringtiefe des Strahls 13 in das Gewebe P verändert werden.

Eine weitere Ausführungsform eines optischen Kohärenztomographen ist in der Figur 5 dargestellt. Hier wird im Tomographen 10 im Gehäuse 50 durch die SLD 52 das Licht erzeugt und tritt dann in einen Phasen-Schmelzkoppler 53 ein. Von dort aus wird es über den Kollimator 54 zum Galvanometerscanner 56 geleitet, aufgeteilt, durch die Linse 55 geführt und als OCT-Strahlen 51 zur Probe P gestrahlt. Die Rückführung erfolgt umgekehrt durch die entsprechenden Bauteile und dann über einen weiteren Kollimator 59, das Gitter 56, die Linse 57 auf den Detektor 58. Mit einer Veränderung des Neigungswinkel des Galvanometerscanner 56 läßt sich die Position des Strahls 51 auf der Oberfläche der Probe P verändern.

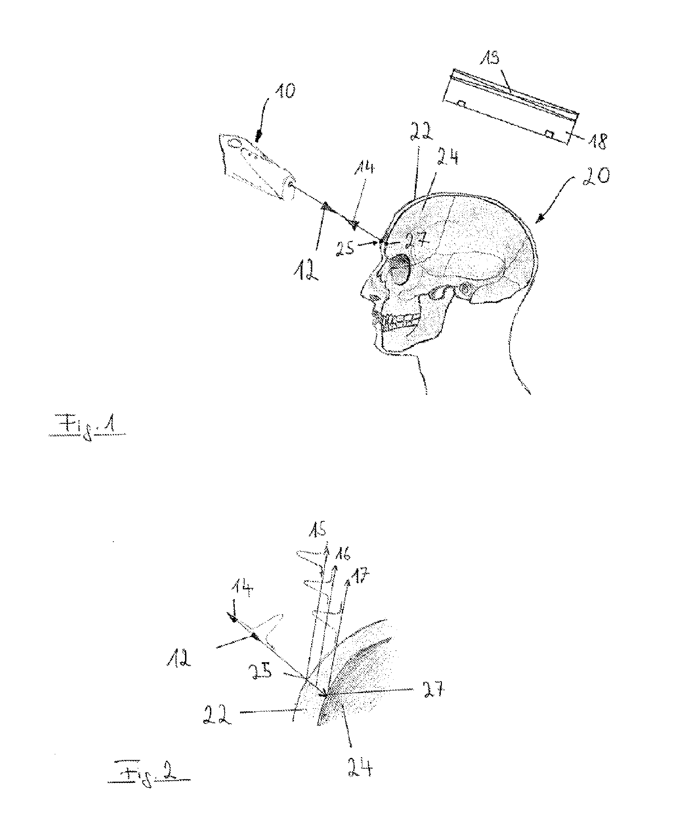

Den Figuren 1 und 2 ist nun die erfindungsgemäße Verwendung in schematischer Form zu entnehmen. Dargestellt ist in der Figur 1 der Kopf 20 eines Patienten, also der Patientenkörperteil, wie er oben bezeichnet wurde. Er besteht vereinfacht aus dem knöchernen Schädel 24 und der darüber liegenden Haut 22, und mit dem Referenztomographen 10 soll nun mit Hilfe des integrierten Navigations- und Trackingsystems 18, 19, das optische Signale positionell erfassen kann, der Kopf 20 des Patienten registriert werden. Dabei wird er einem vorab erstellten Bilddatensatz positionell zugeordnet, der beispielsweise aus einer CT- oder einer MR-Bilderfassung stammt.

Bei der dargestellten Methode der Figur 1 ist der Kohärenztomograph nicht mit einer Referenzanordnung versehen, er ist also frei bewegbar und wird nicht durch das Trackingsystem 18 lokalisiert. Er sendet den OCT-Strahl 12 aus, der auf den Kopf 20 trifft und zurückgestrahlt wird.

Zur Verdeutlichung wird hier auf die Figur 2 Bezug genommen. Hier ist der einfallende Strahl 12 gezeigt, der in verschiedener Weise zurückgestrahlt wird, was hier nur grundsätzlich durch die Rückstrahlen 15, 16 und 17 angedeutet wird. Mit den Strahlen 15, 16 und 17 wird in Figur 2 jeweils ein seitlicher Anteil des Rückstrahls aufgezeigt, um die verschiedenen Reflektionsebenen bzw. Schichten deutlich zu machen. Zur Auswertung bei der Tiefenbestimmung des Referenzpunktes 27 auf der knöchernen Schädelstruktur wird aber natürlich der Rückstrahl 14 im Kohärenztomographen 10 verarbeitet, der entgegen dem OCT-Strahl 12 zurückkehrt (siehe Figur 3). Man sieht aber an der Darstellung der Figur 2, dass der OCT-Strahl 12 in verschiedenen Ebenen unterschiedliche Rückläufer bzw. Reflektionsstrahlen 15, 16 und 17 erzeugt, und hierdurch lässt sich ein Tiefenprofil erstellen. Interessant ist für eine hier gewählte Art der erfindungsgemäßen Registrierung noch speziell der tatsächlich schräg reflektierte Strahl 15, der einen Lichtpunkt 25 auf der Hautoberseite repräsentiert. Dieser Lichtpunkt auf der Hautoberseite kann nämlich vom Trackingsystem 18 erfasst und positionell eingeordnet werden, wodurch man schon einen absoluten Raumpunkt (mit bestimmten Raumkoordinaten) aus dem Trackingsystem erhält, der dann zusammen mit der Tiefeninformation für den Punkt 27 aus der optischen Kohärenztomographie eine sehr gute Positionserfassung und Registrierung zulässt. Der Punkt 25 ist zwar wieder ein Reflektionspunkt auf der Haut, jedoch genügt diese Informationsgenauigkeit, weil die tatsächliche Registrierung auf den "harten" Werten für den knöchernen Schädel und dessen Oberfläche basiert. Ein entsprechendes Registrierungs-Matching von Oberflächen, die durch mehrere OCT-Punkte des Schädelknochens erhalten werden (Punkte 27) wird also die höchste Genauigkeit ergeben, während die Information über die Position des reflektierten Lichtpunkts 25 als Einstiegshilfe für die Zuordnung beim computerunterstützten Matching-Verfahren dient.

Wenn - wie in Figur 1 als Möglichkeit dargestellt - eine Referenzanordnung 13 am Tomographen 10 angeordnet ist, muss nicht unbedingt der "Umweg" über den Lichtpunkt 25 gemacht werden. Man würde dann die Position des Tomographen 10 und damit der Lichtquelle bzw. des Lichtaustrittspunktes kennen und müsste dieser Position nur noch die Tiefeninformation aus der optischen Kohärenztomographie hinzufügen, um direkt den Referenzpunkt 27 lokalisieren zu können.

## Patentansprüche

1. Medizintechnisches Bildregistrierungsverfahren, bei dem ein realer Patientenkörperteil (20) positionell einem gespeicherten Bild des Körperteils (20) computerunterstützt zugeordnet wird, wobei der Patientenkörperteil (20) eine starre Struktur (24), und eine darüber liegende Weichstruktur (22) aufweist, **dadurch gekennzeichnet, dass** bei der Erfassung der realen Raumposition des Körperteils (20) die Position mindestens eines Referenzpunktes (27) auf der starren Struktur (24) durch die geschlossene Weichstruktur (22) hindurch mittels einer optischen Kohärenztomographie (OCT) ermittelt wird.

2. Verfahren nach Anspruch 1, bei dem die reale Raumposition mehrerer Referenzpunkte, einer Wolke von Referenzpunkten, linienförmig aneinander liegender oder beabstandet nebeneinander liegender Referenzpunkte bestimmt wird.

3. Verfahren nach Anspruch 1 oder 2, bei dem die starre Struktur eine knöcherne oder Knorpelstruktur ist.

4. Verfahren nach einem der Ansprüche 1 bis 3, bei dem die Weichstruktur eine Hautstruktur ist.

5. Verfahren nach einem der Ansprüche 1 bis 4, bei dem die Raumpositionserfassung mit Hilfe eines medizintechnischen, optischen Trackingsystems (18) erfolgt und die Registrierung insbesondere durch ein medizintechnisches Navigationssystem (19) durchgeführt wird, dem das Trackingsystem (18) zugeordnet ist.

6. Verfahren nach einem der Ansprüche 1 bis 5, bei dem durch einen optischen Kohärenztomographen (10) oder seine Abstrahleinheit ein OCT-Strahl auf das Patientenkörperteil aufgestrahlt wird.

7. Verfahren nach einem der Ansprüche 1 bis 6, bei dem die Raumlage des Referenzpunktes (27) auf der starren Struktur ermittelt wird durch:
a) die räumliche Erfassung des ihm zugehörigen OCT-Strahl-Reflexpunktes außen auf der Weichstruktur mittels des Trackingsystems, und
b) die Bestimmung der Tiefenlage des Referenzpunktes unter dem Reflexpunkt mittels der optischen Kohärenztomographie.

8. Verfahren nach einem der Ansprüche 1 bis 7, bei dem die Raumlage des Referenzpunktes (27) auf der starren Struktur ermittelt wird durch:
a) die Erfassung der Raumposition des optischen Kohärenztomographen (10) oder seiner Abstrahleinheit, insbesondere durch eine daran angeordnete Referenzeinrichtung bzw. -anordnung (13), mittels des Trackingsystems, und
b) die Bestimmung der Raumlage des Referenzpunktes bezüglich der Raumposition des optischen Kohärenztomographen (10) oder seiner Abstrahleinheit mittels der optischen Kohärenztomographie.

9. Verfahren nach einem der Ansprüche 1 bis 8, bei dem der optische Kohärenztomograph oder seine Abstrahleinheit bei der Positionsermittlung für den Referenzpunkt (27) mit der Weichstruktur (22) in Kontakt ist.

10. Verfahren nach einem der Ansprüche 1 bis 8, bei dem der optische Kohärenztomograph oder seine Abstrahleinheit bei der Positionsermittlung für den Referenzpunkt (27) kontaktlos über der Weichstruktur (22) angeordnet ist.

11. Verfahren nach einem der Ansprüche 1 bis 10, bei dem bei der Registrierung der räumlich erfassten Referenzpunkte mit dem gespeicherten Bild eine Zuordnung diskreter Bildwerte erfolgt, wobei insbesondere erfasste Strukturkanten aufeinander registriert werden.

12. Verfahren nach einem der Ansprüche 1 bis 11, bei dem bei der Registrierung der räumlich erfassten Referenzpunkte mit dem gespeicherten Bild eine Grauwert-Zuordnung der Bildwerte erfolgt.

13. Pointereinheit eines medizintechnischen Bildregistrierungsgeräts, mit dessen Hilfe ein realer Patientenkörperteil (20) positionell einem gespeicherten Bild des Körperteils (20) computerunterstützt zugeordnet wird, wobei mit der Pointereinheit ein Referenzpunkt (27) zur Zuordnung identifiziert bzw. angezeigt wird, **dadurch gekennzeichnet, dass** die Pointereinheit einen optischen Kohärenztomographen (OCT) (10) oder seine Abstrahleinheit umfasst.

14. Pointereinheit nach Anspruch 13, **dadurch gekennzeichnet, dass** sie aus einem optischen Kohärenztomographen (OCT) (10) oder seiner Abstrahleinheit aufgebaut ist.

15. Pointereinheit nach Anspruch 13 oder 14, **dadurch gekennzeichnet, dass** sie mit einer Referenzeinrichtung bzw. -anordnung (13) versehen ist, die von einem medizintechnischen Trackingsystem identifiziert und/oder positionell erfasst werden kann.

16. Pointereinheit nach einem der Ansprüche 13 bis 15, **dadurch gekennzeichnet, dass** sie mit einer Datenübertragungsvorrichtung (31), insbesondere einer kabelgebundenen oder kabellosen Datenübertragungsvorrichtung, versehen ist, um ermittelte Referenzpunkt-Positionsdaten an eine Auswerteeinheit, insbesondere ein zugeordnetes medizintechnisches Navigationssystem (19), zu übermitteln.

17. Pointereinheit nach einem der Ansprüche 13 bis 16, **dadurch gekennzeichnet, dass** die Abstrahleinheit des optischen Kohärenztomographen einen Aufsatz aufweist, insbesondere eine verlängerte Optik, speziell eine Lichtleiteroptik, wobei die Lichtleiteroptik im Besonderen ein starrer, länglicher Verlängerungsaufsatz ist.

## Claims

1. A medical image registration method in which an actual part (20) of a patient's body is locationally assigned to a stored image of the part (20) of the body with computer assistance, wherein the part (20) of the patient's body comprises a rigid structure (24) and a soft-tissue structure (22) above it, **characterised in that** the location of at least one reference point (27) on the rigid structure (24) is ascertained, through the closed soft-tissue structure (22), by means of optical coherence tomography (OCT), when detecting the actual spatial location of the part (20) of the body.

2. The method according to claim 1, wherein the actual spatial location of a plurality of reference points, a scatterplot of reference points, linearly adjacent reference points or adjacently spaced reference points is determined.

3. The method according to claim 1 or 2, wherein the rigid structure is an osseous structure or a cartilage structure.

4. The method according to any one of claims 1 to 3, wherein the soft-tissue structure is a skin structure.

5. The method according to any one of claims 1 to 4, wherein the spatial locations are detected with the aid of a medical, optical tracking system (18), and registration is in particular performed by a medical navigation system (19) which the tracking system (18) is assigned to.

6. The method according to any one of claims 1 to 5, wherein an OCT beam is radiated onto the part of the patient's body by an optical coherence tomography scanner (10) or its emitting unit.

7. The method according to any one of claims 1 to 6, wherein the spatial position of the reference point (27) on the rigid structure is ascertained by:
a) spatially detecting the OCT beam reflection point corresponding to it on the outside of the soft-tissue structure, by means of the tracking system; and
b) determining the depth position of the reference point beneath the reflection point, by means of optical coherence tomography.

8. The method according to any one of claims 1 to 7, wherein the spatial position of the reference point (27) on the rigid structure is ascertained by:
a) detecting the spatial location of the optical coherence tomography scanner (10) or its emitting unit, in particular using a reference device and/or reference array (13) arranged on it, by means of the tracking system; and
b) determining the spatial position of the reference point with respect to the spatial location of the optical coherence tomography scanner (10) or its emitting unit, by means of optical coherence tomography.

9. The method according to any one of claims 1 to 8, wherein when ascertaining the location of the reference point (27), the optical coherence tomography scanner or its emitting unit is in contact with the soft-tissue structure (22).

10. The method according to any one of claims 1 to 8, wherein when ascertaining the location of the reference point (27), the optical coherence tomography scanner or its emitting unit is arranged without contact above the soft-tissue structure (22).

11. The method according to any one of claims 1 to 10, wherein when registering the spatially detected reference points to the stored image, discrete image values are assigned, wherein detected structure edges are in particular registered onto each other.

12. The method according to any one of claims 1 to 11, wherein when registering the spatially detected reference points to the stored image, grey values of the image values are assigned.

13. A pointer unit of a medical image registration apparatus, with the aid of which an actual part (20) of a patient's body is locationally assigned to a stored image of the part (20) of the body with computer assistance, wherein the pointer unit identifies and/or indicates a reference point (27) for assignment, **characterised in that** the pointer unit comprises an optical coherence tomography (OCT) scanner (10) or its emitting unit.

14. The pointer unit according to claim 13, **characterised in that** it is composed of an optical coherence tomography (OCT) scanner (10) or its emitting unit.

15. The pointer unit according to claim 13 or 14, **characterised in that** it is provided with a reference device and/or reference array (13) which can be identified and/or locationally detected by a medical tracking system.

16. The pointer unit according to any one of claims 13 to 15, **characterised in that** it is provided with a data transfer device (31), in particular a cable-connected or wireless data transfer device, in order to transfer ascertained reference point location data to an evaluating unit, in particular an assigned medical navigation system (19).

17. The pointer unit according to any one of claims 13 to 16, **characterised in that** the emitting unit of the optical coherence tomography scanner comprises an attachment, in particular an extended lens system, specifically an optical fibre lens system, wherein the optical fibre lens system is in particular a rigid, elongated extending attachment.

## Revendications

1. Procédé de recalage d'image, dans lequel la position d'une partie réelle du corps d'un patient (20) est attribuée à l'aide d'un ordinateur à une image enregistrée de la partie du corps (20), où la partie du corps du patient (20) comporte une structure rigide (24) et une structure molle (22) située pardessus, **caractérisé en ce que** lors de la détection de la position réelle de la partie du corps (20) dans l'espace, la position d'au moins un point de référence (27) sur la structure rigide (24) est déterminée à travers la structure molle (22) fermée au moyen d'une tomographie en cohérence optique (TCO).

2. Procédé selon la revendication 1, dans lequel est déterminée la position réelle de plusieurs points de référence dans l'espace, d'un nuage de points de référence, de points de référence disposés l'un contre l'autre de manière linéaire ou de points de référence disposés l'un à côté de l'autre de manière espacée.

3. Procédé selon la revendication 1 ou 2, dans lequel la structure rigide est une structure osseuse ou une structure cartilagineuse.

4. Procédé selon l'une des revendications 1 à 3, dans lequel la structure molle est une structure épidermique.

5. Procédé selon l'une des revendications 1 à 4, dans lequel la détection des positions dans l'espace s'effectue à l'aide d'un système de suivi et de localisation optique médico-technique (18) et le recalage s'effectue en particulier via un système de navigation médico-technique (19) auquel est assigné le système de suivi et de localisation (18).

6. Procédé selon l'une des revendications 1 à 5, dans lequel un rayon TCO est irradié sur la partie du corps du patient par un appareil de tomographie en cohérence optique (10) ou par son unité d'irradiation.

7. Procédé selon l'une des revendications 1 à 6, dans lequel l'orientation spatiale du point de référence (27) sur la structure rigide est déterminée par :
a) la détection dans l'espace du point de réflexion du rayon OCT lui correspondant sur l'extérieur de la structure molle au moyen du système de suivi et de localisation, et
b) la détermination de la profondeur du point de référence sous le point de réflexion au moyen de la tomographie en cohérence optique.

8. Procédé selon l'une des revendications 1 à 7, dans lequel l'orientation spatiale du point de référence (27) sur la structure rigide est déterminée par :
a) la détection de la position dans l'espace de l'appareil de tomographie en cohérence optique (10) ou de son unité d'irradiation, en particulier par un dispositif ou un système de référence (13) au moyen du système de suivi et de localisation, et
b) la détermination de l'orientation spatiale du point de référence par rapport à la position dans l'espace de l'appareil de tomographie en cohérence optique (10) ou de son unité d'irradiation au moyen de la tomographie en cohérence optique.

9. Procédé selon l'une des revendications 1 à 8, dans lequel l'appareil de tomographie en cohérence optique ou son unité d'irradiation sont en contact avec la structure molle (22) lors de la détermination de la position du point de référence (27).

10. Procédé selon l'une des revendications 1 à 8, dans lequel l'appareil de tomographie en cohérence optique ou son unité d'irradiation est disposé sans contact au-dessus de la structure molle (22) pour la détermination du point de référence (27).

11. Procédé selon l'une des revendications 1 à 10, dans lequel est effectuée une attribution de valeurs discrètes d'images lors du recalage des points de référence détectés dans l'espace avec l'image enregistrée, où sont notamment recalées les unes sur les autres des arrêtes de structure détectées.

12. Procédé selon l'une des revendications 1 à 11, dans lequel est effectuée une attribution des niveaux de gris des valeurs d'image lors du recalage des points de référence détectés dans l'espace avec l'image enregistrée.

13. Unité de pointage d'un appareil de recalage d'image médico-technique, à l'aide de laquelle la position d'une partie du corps d'un patient (20) est attribuée à l'aide d'un ordinateur à une image enregistrée de la partie du corps (20), où un point de référence (27) est identifié ou affiché avec l'unité de pointage, **caractérisée en ce que** l'unité de pointage comprend un appareil de tomographie en cohérence optique (TCO) (10) ou son unité d'irradiation.

14. Unité de pointage selon la revendication 13, **caractérisée en ce qu'**elle est composée d'un appareil de tomographie en cohérence optique (OCT) (10) ou de son unité d'irradiation.

15. Unité de pointage selon la revendication 13 ou 14, **caractérisée en ce qu'**elle est équipée d'un dispositif ou d'un système de référence (13) pouvant être identifiée ou dont la position peut être détectée par un système de suivi et de localisation médico-technique.

16. Unité de pointage selon l'une des revendications 13 à 14, **caractérisée en ce qu'**elle est équipée d'un dispositif de transmission de données (31), en particulier d'un dispositif de transmission de données avec ou sans fil, afin de transmettre des données de position de points de référence à une unité de traitement, en particulier à un système de navigation médico-technique (19) attribué.

17. Unité de pointage selon l'une des revendications 13 à 16, **caractérisée en ce que** l'unité d'irradiation de l'appareil de tomographie en cohérence optique comporte un embout, en particulier une optique, plus spécifiquement une optique à fibre optique, où l'optique à fibre optique est en particulier un embout de prolongement rigide allongé.
